**Europäisches Patentamt**

(19) **European Patent Office**

**Office européen des brevets**

(11) Publication number : **0 514 096 A2**

(12) **EUROPEAN PATENT APPLICATION**

(21) Application number : **92304180.0**

(22) Date of filing : **08.05.92**

(51) Int. Cl.⁵ : **A61L 27/00**

(30) Priority : **13.05.91 US 698856**

(43) Date of publication of application :
**19.11.92 Bulletin 92/47**

(84) Designated Contracting States :
**AT BE CH DE DK ES FR GB IT LI NL SE**

(71) Applicant : **IOPTEX RESEARCH INC.**
**15715 Arrow Highway**
**Irwindale, California 91706-2094 (US)**

(72) Inventor : **Gupta, Amitava**
**1606 Virginia Road**
**San Marino, California 91108 (US)**

(74) Representative : **Cole, William Gwyn, Dr.**
**Smith & Nephew p l c Corporate Patents and**
**Trade Marks Dept. Gilston Park**
**Harlow Essex CM20 2RQ (GB)**

(54) **Deformable-elastic intraocular lens.**

㉗ A deformable-elastic intraocular lens comprising a deformable-elastic lens body of crosslinked acrylic material formed of copolymers of methacrylate and acrylate esters which are relatively hard and relatively soft at body temperature, crosslinked with a diacrylate ester to produce an acrylic copolymer having a substantially tack-free surface, a crosslink density of between $1.2 \times 10^{-1}$ and $3.0 \times 10^{-1}$ moles per liter, and a glass transition temperature in the range of -30°C to 25°C, a tensile modulus between 1000 and 3000 psi and an elongation a break of 100% or greater.

EP 0 514 096 A2

## BACKGROUND OF THE INVENTIONS

The present invention relates generally to improvements in intraocular lenses (IOLs) designed for surgical implantation into the eye, for example, as a replacement for a cataractous or injured natural lens. More specifically, the invention relates to improvements in deformable IOLs which can be folded or rolled to a relatively low profile size to fit into the eye through a relatively small incision, and then within the eye naturally return to an initial nondeformed shape with predetermined optical properties.

IOLs are well known in the art for implantation into the eye as a replacement for a natural crystalline lens which has been surgically removed typically due to opacification, commonly referred to as a cataract condition. Such IOLs have been formed from a small disk of transparent glass or plastic material having appropriately shaped lens surfaces to achieve a desired set of optical properties. The IOL is implanted directly into the eye, typically after removal of the natural crystalline lens, via an incision formed in ocular tissue such as the sclera outside the normal line of sight. Many IOLs are designed for implantation into the so-called posterior chamber of the eye behind the iris and pupil, whereas other IOLs are adapted for placement into the anterior chamber in front of the iris and pupil. In most IOL designs, support structures are attached to or formed intergrally with a central lens body or optic and project outwardly therefrom to contact eye tissue at the periphery of the posterior or anterior chamber, thereby retaining the lens body or optic in generally centred relation with the line of sight passing through the pupil.

In the past, most IOLs have been formed from polymethylmethacrylate (PMMA) which is relatively light in weight, possesses excellent optical properties, and is generally considered to be relatively inert when implanted into the eye, thereby avoiding adverse tissue reactions. However, PMMA comprises a plastic matrix which, when formed into the shape of a lens, possesses high rigidity and cannot be deformed by folding, rolling, compression, etc. Accordingly, the use of PMMA lenses requires a relatively large incision in the ocular tissue sufficient to accommodate the entire diametric size of the lens body; which is typically six millimetres or larger, together with the accompanying lens support structures. Although resilient lens support structures such as polypropylene loops or haptics are commonly used and advantageously may be folded over the lens body during insertion, such resilient haptics are anchored into the periphery of the hard plastic lens body and thus tend to spring back to their initial unfolded shape with a rapid snap like action during IOL implantation, resulting in undesired trauma to sensitive eye tissues.

While IOLs with rigid PMMA lens bodies have gained widespread acceptance and use, it has been recognized that deformable IOLs have the potential of providing medical benefits well beyond those associated with current IOLs including right lens bodies. More particularly, an IOL including a deformable transparent lens body which may be folded or rolled into a reduced profile size may fit through a relatively small incision in ocular tissue and after insertion and release within the eye return to its original size and shape by virtue of its natural resilience. The use of a smaller incision would beneficially result in a safer overall surgical procedure requiring fewer stitches and reduced likelihood of postoperative complications such as infections. In addition, a small incision would reduce the incidence of postoperative astigmatism and substantially reduce rehabilitation time. Second, it is anticipated that IOLs with deformable lens bodies may reduce the potential for complications secondary to contact or rubbing against delicate uveal tissues. Also, deformable IOLs may decrease the potential for pigmentary dispersion or pigmentary glaucoma. Finally, it is anticipated that deformable IOLs will provide an added margin of safety for patients with blood dyscarsias, coagulopalthies and hematologic matogrant disease as well as those patients being given anti-coagulant therapy.

Accordingly, deformable IOLs formed of silicones and hydrogels have been proposed for implantation. For example, in 19983, Fyodorov reported on chemical testing of a silicone IOL (Fyodorov, S.W. et al "Initial Clinical Testing of a Silicone Intraocular Lens" Interzonal Scientific/Practical Conference of Ophthalmologists of Western and Eastern Siberia and the Far East, Conference proceedings 4: 22-24, 1983, Vladivostock). Also in 1983, Mazzacco and Davidson presented initial data on the implantation of silicone IOLs with 6 mm optical zones through 3 mm incisions (Mazzacco, T.R. and Davidson, V.A. "6 mm Optic for a 3 mm Wound" presented at the A.I.O.I.S. United States Intraocular Lens Symposium, New Orleans, Louisiana, March 1983). Wichterle and his associates developed a hydrogel of hydrophilic polyacrylates for orbital and intracameral implants in 1960 while Epstein implanted flexible IOLs comprises of poly(hydro hydroxyethyl methacrylate) in 1976 and 1977. The condition of some patients implanted with such lens was followed until 1984 ("Insertion Techniques and Clinical Experience with HEMA Lenses" Soft Implant Lenses in Cataract Surgery T.R. Mazzacco, G.M. Rajaciach, E. Epstein, published by Slack Inc., 1986, pp. 11).

Unfortunately, silicones and hydrogels have several well documented deficiencies which hinder their use as IOL materials. In particular, silicones cause complement activation leading to the production of C-4 proteins, a symptom of bio-incompatibility. Also, while silicones may be folded, when released they tend to snap back or regain their unfolded shape too rapidly, posing a threat to the integrity of the endothelial cell layer of the eye.

In addition, the long term stability of UV-absorbing silicone formulations is uncertain. As for hydrogels, it has been found that hydrogel materials when hydrated vary in composition including water content from lot to lot. Such variability induces a corresponding variability in the refractive power of IOL lens bodies formed of hydrogel material. Therefore, hydrogel IOLs need to be hydrated in order to determine their refractive power in an implanted state. Unfortunately, hydrated lenses cannot be safely stored in the wet state without losing sterilization. If they are dehydrated subsequently, the process of hydrothermal cycling reduces the tensile strength of the IOL material and may cause cracks or crazes to develop in the lens body.

Other deformable IOLs have been described in United States Patents 4,573,998 and 4,608,049. More specifically, the '998 patent is directed to methods for implantation of deformable IOLs. The patent describes an IOL having an optical zone portion composed of materials such as polyurethane elastomers, silicone elastomers, hydrogel polymer collagen compounds, organic or synthetic gel compounds and combinations thereof. In practice, such materials possess the disadvantages previously attributed to silicone and hydrogel materials.

The '049 patent describes two basic types of deformable IOLs. The first type includes a lens body of one or more rigid portions hinged or otherwise connected to overlap each other when it is desired to reduce the profile of the lens body as during implantation of the lens. Such lens configuarations are difficult to construct and to manipulate during implantation and further suffer from the limitations associated with rigid IOLs. The second type of IOL described by the '049 patent includes a deformable lens body characterized as being capable of return to an undeformed configuration after insertion into the eye. The lens body may be of silicone rubber or an acrylate polymer with ethylene glycol dimethacrylate as a crosslinking agent producing a material of a rubber consistency. The deformable lens body is secured to an L-shaped fixation member around which it may be curled during insertion into the eye. The silicone rubber IOL of the '049 patent suffers from the limitations previously attributed to silicone IOLs. The acrylate polymer lens body described in the '049 patent is a hydrogel of a relatively hard consistency (subject to the foregoing problems attributed to hydrogels) while other acrylate polymers known to be pliable are prone to mechanical failure upon compression or folding and are subject to degratation in the eye.

In view of the foregoing, it is apparent that there is a need for an intraocular lens and lens material having an improved balance of superior optical characteristics, flexibility, elasticity, elastic memory, and tensile strength. The present invention satisfies such needs.

Generally speaking, the present invention comprises an IOL having a deformable-elastic transparent lens body of crosslinked acrylic material having a tensile strength sufficient to resist deformation after implantation into the eye as by forces exerted by growing tissue around the IOL; a flexibility as measured by elongation at break sufficient to allow the lens body to be readily folded, rolled or otherwise deformed to a low profile condition for implantation through a small incision into the eye; an elastic memory which enables the folded lens body to naturally and at a controlled rate return to its original shape and optical resolution without damaging or otherwise traumatizing eye tissue; and low-tack surface which will not stick to surgical instruments used to hold and guide the lens body during insertion and positioning within the eye. In particular, the crosslinked acrylic material comprises copolymers of methacrylate and acrylate esters which are relatively hard and relatively hard at body temperature, crosslinked with a diacrylate ester to produce an acrylic material having a substantially tack-free surface, a crosslink density between $1.2 \times 10^{-1}$ moles per liter, a glass transition modulus in the range of 70.3 to 210.9 kg cm$^{-2}$ (1000 to 3000 psi) and an elongation at break of between 100 and 300%. Such a lens body is easily folded, rolled or otherwise deformed into a low profile for insertion through a small incision and after insertion will naturally return to its original optical resolution at a slow controlled rate in between 20 and 190 seconds even if the lens body has been deformed to a low profile condition for an extended period of time. The slow return allows the surgeon adequate time to locate the folded IOL in the eye before the lens body returns to its original shape and resolution and insures that the unfolding of the lens will not damage or otherwise traumatize ocular tissue. Furthermore, a lens body of the foregoing material and composition possesses a desired tensile strength to resist deformation in response to forces exerted by tissue growing around the implanted lens body thereby maintaining the desired optical characteristics and resolution of the lens body.

Preferably, in the formation of the deformable-elastic acrylic material, the copolymers of methacrylate and acrylate esters are mixed at approximately a 45 to 55 weight percent ratio and the relatively hard methacrylate ester is a fluoracrylate. The fluoroacrylate functions as a surface energy lowering agent as well as a monomer providing long term stable inertness and tensile strength to the polymer without adversely effecting the pliancy of the resulting material. In this regard, the fluoroacrylate is present in a concentration range by weight of between 5 and 25% and preferably is trifluoro ethyl methacrylate. Also in the preferred formulation of the crosslinked acrylic, the mixture of the copolymers is partially polymerized prior to chemical crosslinking with diacrylate ester in a concentration range of range 0.5 and 3.0 percent by weight.

The resulting crosslinked acrylic material may be molded and formed into lens bodies machined to have the desired optical characteristics and resolution with haptics extending therefrom either integral with or separately attached to the lens body. Preferably, the crosslinked acrylic material formed according to the present invention is machined and otherwise processed at low temperatures in the range of -80 to -10°C and preferably at -60°C. In particular, during cutting the lens body is maintained at a temperature below its Beta-relaxation temperature where the material is even harder than at is glass transition temperature.

The accompanying drawings illustrate the invention. In such drawings:

FIGURE 1 is a front elevation view of an exemplary IOL formed in accordance with the novel features of the invention;

FIGURE 2 is a side elevation view of the IOL depicted in FIGURE 1;

FIGURE 3 is a fragmented front elevation view depicting the IOL of FIGURE 1 implanted in to the posterior chamber of an eye;

FIGURE 4 is an enlarged perspective view illustrating the lens of FIGURE 1 rolled into a reduced size profile prior to implantation;

FIGURE 5 is a fragmented sectional view illustrating implantation of the lens into the posterior chamber of the eye;

FIGURE 6 is a graphic representation of the relative stiffness of the body of the IOL as a function of temperature;

FIGURE 7 is a flow diagram in block form illustrating a preferred form of a method for producing a deformable-elastic acrylic material comprising a lens body of an IOL in accordance with the present invention;

FIGURES 8A & 8B illustrate two molds useful in the method of the present invention for forming acrylic material into intraocular lens bodies; and

FIGURE 9 is a plan view of a bottom part of a mold useful in forming a one piece IOL in accordance with the present invention. FIGURE 9 also illustrates a plan view of the part produced from such a mold.

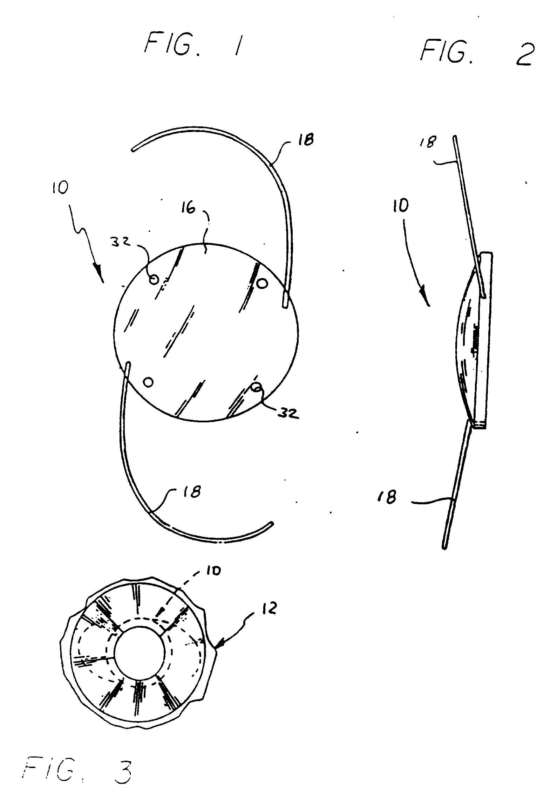

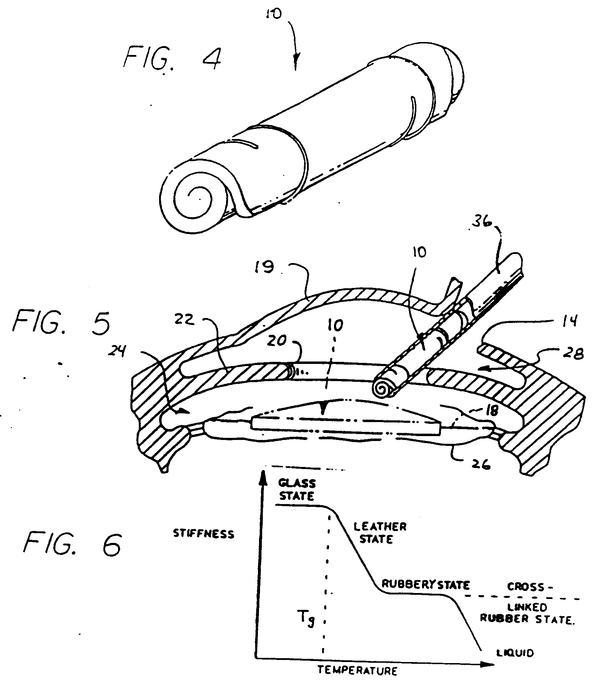

As shown in the exemplary drawings, one preferred form of an improved IOL is referred to generally by the reference number 10 in FIGURES 1-5. The improved lens 10 is deformable (FIGURES 4 and 5) to a reduced profile size to permit implantation into an eye 12 through a relatively small incision 14. The lens 10 is formed with a selected set of physical characteristics to expand within the eye slowly but substantially completely to its initial nondeformed state and optical resolution without trauma to delicate eye tissue.

As shown in Figures 1, 2 and 3, the IOL 10 of the present inventions comprises a traditional disk-shaped lens body 16 having an appropriate diametric size typically on the order of about six millimeters and a combination of surface shapes on the anterior-posterior sides to provide selected dioptric characteristics, with a convexo-plano shape being shown by way of example in the illustrative drawings. The IOL is adapted for implantation into the eye 12 subsequent to surgical removal of the natural crystalline lens, typically due to a cataract condition. Alternately, if desired, the IOL can be implanted to obtain refractive correction of the natural lens. Support structures such as a pair of outwardly radiating and curved resilient loops or haptics 18 are secured to the lens body 16 and function to support the lens body within the eye 12, as will be described in more detail. The haptics 18 may be anteriorly angulated as shown in FIGURE 2, and/or provided in other configurations such as a trio of loops or alternate support structures formed integrally with the lens body, in accordance with the particular intraocular lens design.

In accordance with known intraocular lens implantation techniques, the IOL 10 is adapted for implantation into the eye through an incision 14 formed in the ocular tissue at a position removed from a normal sight line passing through the transparent cornea 19, as viewed in FIGURE 5, and further through the pupil 20 defined by the iris 22. The IOL 10 can be designed as shown in FIGURE 5 for implantation through the pupil 20 into the so-called posterior chamber 24 behind the iris 22, typically within a capsular bag 26 which has bee anteriorly ruptured in the course of extracapsular extrusion of the natural crystalline lens. Alternately, if desired, the IOL 10 can be implanted into the anterior chamber 28 at the front side of the iris 22. In either case, support structures such as the illustrative paid of outwardly curving support loops 18 seat against surrounding tissue at the chamber periphery to retain the lens body 16 generally centered on the normal line of sight. Positioning holes 32 may also be provided near the periphery of the lens body 16 and are easily engaged by appropriate surgical instruments (not shown) to facilitate lens manipulation by the surgeon to the desired position within the eye.

In accordance with primary aspects of the invention, the lens body 16 of the IOL 10 is formed from a deformable-elastic transparent crosslinked acrylic material with a unique balance of flexibility, elasticity, tensile strength and softness properties yielding significant advantages during implantation and subsequent use. More specifically, because of its improved flexibility, the IOL is capable of being reduced in profile size to fit through the incision 14 of reduced size in comparison with conventional hard plastic lens of polymethylmethacrylate (PMMA) or the like. Because of its controlled elasticity, the lens body 16 anchors the haptics 18 with sufficient damping to prevent rapid or snap-action movement of the haptics 18 toward their normal unstressed configurations, thereby preventing the haptics from sharply striking and damaging eye tissue. Moreover, the lens body possess a relatively slow speed of return or retraction of about twenty (20) to one-hundred eighty (180) seconds from a deformed state as shown in Figure 4 to its initial undeformed state to avoid striking and damaging eye tissue. Further, the lens body has excellent elastic memory to insure substantially complete return to the undeformed state without plastic deformation in the form of fold lines or creases or other plastic deformation in the form of fold lines or creases or other distortions which would otherwise impair optical quality.

The preferred crosslinked acrylic material for the IOL 10 comprises copolymers or methacrylate and acrylate esters which are relatively hard and relatively soft at body temperature, partially polymerized, chemically crosslinked with a diacrylate ester and cured. The resulting acrylic has relatively leathery characteristic at temperature conditions corresponding with or approximating body temperature. More specifically, with reference to FIGURE 6, the crosslinked acrylic composition is selected to have a glass transition temperature (Tg) somewhat below body temperature so that the lens will exhibit a stiffness (Young's modulus) at a body temperature environment reflecting a relatively leathery characteristic. In addition, the crosslinked acrylic composition is chosen to have highly elastic or viscoelastic properties with substantially no plastic deformation and a relatively slow speed of retraction. With such a combination of characteristics, the IOL 10 can be deformed as by rolling upon itself together with the haptics 18 as viewed in FIGURES 4 and 5 for facilitated implantation via a small insertion tube 36 passed through the small incision 14. In particular, the hollow insertion tube 36 may be prefilled with Healon or the like for lubrication purposes. The IOL 10, including the lens body 16 and haptics 18, may be temperature prepared in advance substantially at body temperature, at which time the IOL 10 and tube 36 within the eye. The thus-released lens is allowed to return to its initial nondeformed state. Importantly, this return movement takes place slowly with excellent elastic memory over a time of at least about twenty seconds. When the lens is substantially completely expanded, the lens position within the eye can be manipulated with appropriate instruments engaging, for example, the positioning holes 32 after which the incision is closed to complete the procedure.

The preferred lens body composition is prepared by copolymerization of transparent acrylic and methacrylic monomers which otherwise exhibit relatively hard and relatively soft physical characteristics in a body temperature environment and a glass transition temperature (Tg) within the range of about -30 to about 25°C and more preferably 0°C. Preferably, the monomers include a fluoromonomer for enhancing the tack-free inertness and tensile strength characteristics of the lens body within the eye and the resulting acrylic is produced by chemical crosslinking with a diacrylate ester to form a stable interpenetrating polymer network having the desired elasticity and elastic memory characteristics.

The following chart lists various monomers which after purification, as by vacuum distillation, may be used in preparing the desired copolymer of crosslinked acrylic material as well as the concentration ranges for such monomers in percent by weight and preferred compositions I and II in percent by weight composition.

| Monomer | Concentration Range | Preferred Compositions % | |
|---|---|---|---|
| | | I | II |
| Ethyl Methacrylate | 25 – 45 | 34 | 34 |
| Trifluoro Ethyl Methacrylate | 5 – 25 | 10 | 10 |
| n-Butyl Acrylate | 30 – 60 | 52 | 0 |
| Ethyl Acrylate | 30 – 60 | 0 | 50.5 |
| 2-Ethyl Hexyl Acrylate | 30 – 60 | 1.5 | 1.5 |
| 2-Hydroxy 4-Ethyloxy-Acryloxy Benzophenone (UV-2098) | 0 – 10 | 1.5 | 1.5 |
| 2, 5 Dimethyl-2,5 Bio (2-Ethyl Hydroxyl Droxyl) Hexane (USP 245) | 0.05 – 0.2 | 0.15 | 0.15 |
| Ethylene Glycol | 2.5-6.0 | 2.5 | 4.0 |

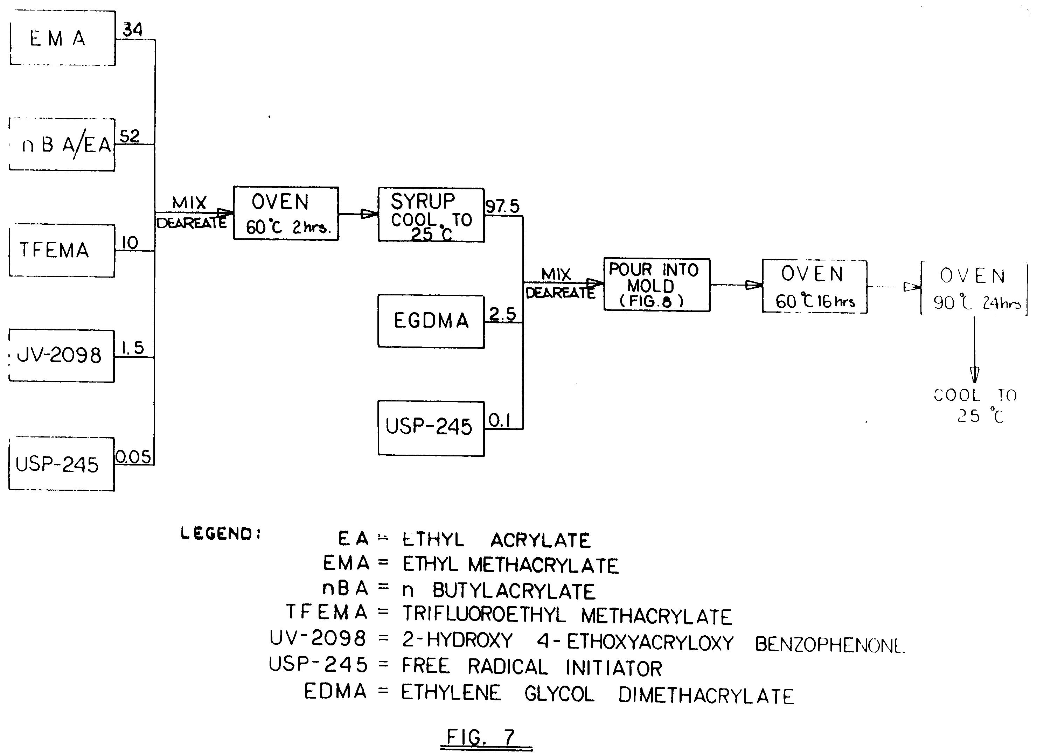

A preferred form of the method for forming the copolymer is depicted in FIGURE 7. As there represented, ethyl methacrylate is mixed with n-butyl acrylate or ethyl acrylate preferably in a weight percent concentration of 34% to 52% respectively. In addition to the methacrylate and acrylate esters of ethyl methacrylate and n-butyl acrylate or etyhl acrylate, the mixture includes 10% by weight of a fluoroacrylate functioning as a surface energy lowering agent. Such fluoroacrylates may be perfluoro octal methacrylate or more preferably trifluorethyl methacrylate. In the mixture, the n-butyl acrylate or ethyl acrylate provides flexibility in the presence of methacrylate esters principally because of the low glass transition temperature thereof. However, the n-butyl acrylate or ethyl acrylate renders the mixture tacky or sticky. Such tackiness is minimized by the fluoroacrylate particularly trifluoroethyl methacrylate. In addition to the foregoing, and as represented in FIGURE 7, the mixture includes a UV-absorber, UV-2098 and a free radical initiator, preferably USP 245, which is one in a class of aliphatic peroxides. The UV-absorber and initiator are present at 1.5 and 0.05% by weight concentrations. The combination is mixed, deareated and placed in an over at about 60°C for two hours. The mixture undergoes partial polymerization to form a viscous syrupy liquid when cooled to about 25°C. The viscous syrupy liquid may be stored for several days at -15°C for subsequent mixing with a crosslinking agent and free radical initiator.

An alternate method of preparing the syrup is to dissolve low molecular weight (number average molecular weight between 30,000-50,000) polymers such as poly(ethyl methacrylate) and poly(n-butyl acrylate) in the same relative concentrations at a polymer - monomer ration ranging from 1:5 to 1:3. The syrup may be filtered through 0.2 micro filter immediately prior to use.

Again, as represented in FIGURE 7, the crosslinking agent may consist of ethylene glycol dimethacrylate. Alternatively, the crosslinking agent may be propylene glycol dimethacrylate or ethylene glycol diacrylate. In each case, the crosslinking agent is mixed in a weight percent concentration of about 2.5 to produce a crosslinked density for the resulting copolymer in a range of $1.2 \times 10^{-1}$ to $3.0 \times 10^{-1}$ moles per liter. Such a crosslinking density provides the resulting polymer with the desired elastic memory and elasticity. In particular, upon being folded, the resulting lens bodies 16 will return to its initial state naturally in about 20 to 180 seconds and preferably about 30 seconds.

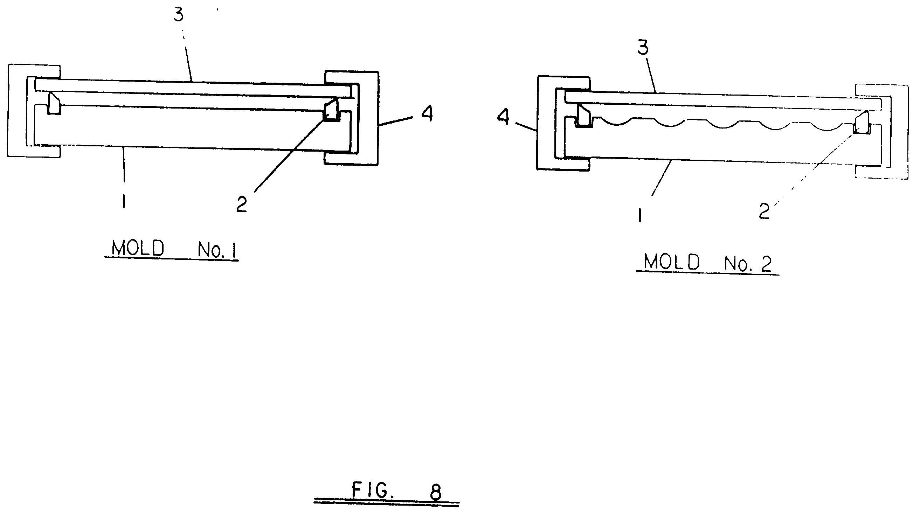

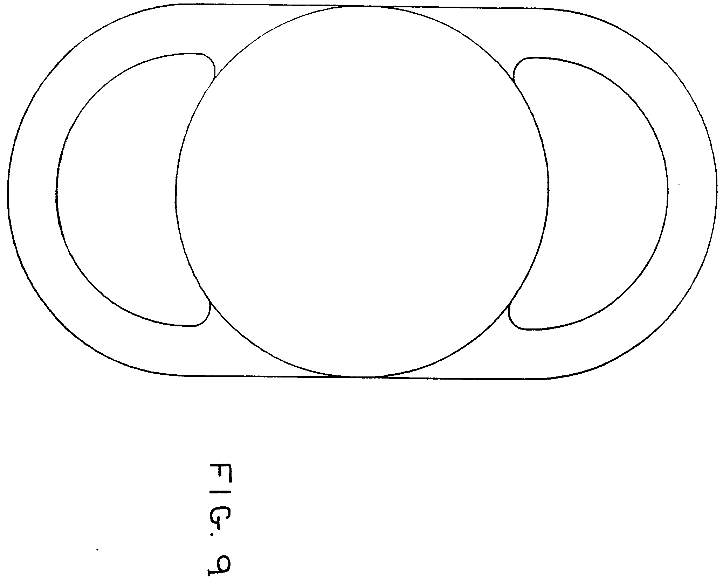

To produce an IOL 10 with the lens body 16 having the foregoing characteristics, and as further depicted in FIGURES 7, 8A and 8B, the syrup, crosslinking agent and initiator (in the indicated percent by weight concentrations) are mixed, deareated and the resulting mixture poured into a mold such as old number 1 or 2 illustrated in FIGURES 8A & 8B or the mold illustrated in FIGURE 9. With respect to molds of FIGURE 8, the resulting mixture is poured onto an aluminium plate 1 bounded by rubber gaskets 2. A glass plate 3 is placed on top of the rubber gaskets and the combination clamped together by clamps 4. The mold is placed in an oven, heated to about 60°C and cured for about 16 hours. The mold is then post cured at about 90°C for 24 hours.

After curing, the mold is disassembled and the sheets formed therein made ready for cutting into cylindrical lens blanks in the case of mold number 1 or deflashing into lens bodies in the case of mold number 2. Alternatively, the mold bottom shown in FIGURE 9 may be used. As illustrated, the mold has slots machined into is aluminium base to accommodate the haptics at an appropriate angle. The molded part from the mod of FIGURE 9 comprises the optic and the haptic elements encased in a thin sheet of flash which may be machined off to produce the finished IOL.

Such cutting and machining to produce the desired IOL may involve conventional miling and lathe techniques with the exception that the part is held at a temperature well below room temperature and preferably between -80 and -10°C. Specifically, it is desired that the material be held below its Beta-relaxation temperature during cutting. Preferably, during cutting, the low temperature environment is formed by exposing the part to a liquid nitrogen spray which maintains the part within the desired temperature range and provides the desired moisture for the cutting operation. As previously noted, at or below its Beta-relaxation temperature, the copolymer material possesses a particularly hard characteristic suitable for high speed and efficient cutting.

An example of a procedure used to fabricate a multi-piece IOL as shown in FIGURE 1 including separate haptics is as follows. First, flat sheets of the crosslinked acrylic are molded at a thickness of between 2 mm and 8 mm as described above and mounted on holders. The material is then cut into disks which are lathe cut at the low temperatures previously described to form the curved planar surfaces and edge cut. The resulting lens bodies are soaked in Freon and chlorofluoro hydrocarbon solven for 20 minutes and then dried for 30 minutes in a vacuum oven at 60°C. The curved surfaces of the lens bodies are then polished at a low temperature. Next, the lens bodies are mounted for drilling of the positioning holes 32 as well as the edge holes for receiving the haptics 18. The positioning holes are typically 0.3 mm while the edge holes for receiving the haptics are typically 0.1 mm in diameter. To mount the haptics into the edge holes, the haptics are located in a stainless needle and one end of the haptic melted to form a thickened blunt tip. The needle is then inserted into the edge hole to force the blunt end of the haptic into the hole at room temperature. The needle is carefully withdrawn allowing the walls of the edge hole to collapse back to their normal position clamping the haptic in place. This operation is then repeated for the other haptic.

Alternatively, for lens bodies molding using mold number 2 illustrated in FIGURES 8A & 8B, the sheet is cored in the area of the lens bodies to cut the lens bodies from the sheet. The resulting lens bodies are then mounted in suitable holders and the foregoing procedure repeated.

Finally, for parts molded from the mold illustrated in FIGURE 9, the flash may be removed on a mill to form the desired one piece IOL.

From the foregoing, it should be appreciated that the IOLs of the present invention may be provided in various geometries adapted for folding or rolling, etc. to a reduced profile configuration thereby permitting implantation into the eye through an incision of reduced size. Within the eye, the deformed lens returns to its original nondeformed state. However, according to the invention, the lens is formed from a material having a combination of excellent elastic memory and slow speed of retraction characteristics. The lens thus returns slowly to the nondeformed state without injuring eye tissue while achieving the final nondeformed state without creases, wrinkles, or other structural deviations which would otherwise result in optical distortions.

A variety of further modifications and improvements to the invention described herein are believed to be apparent to those skilled in the art. Accordingly, no limitation is intended by way of the description herein, except as set forth in the appended claims.

## Claims

1. A deformable-elastic intraocular lens (IOL) body, comprising:

   a deformable-elastic lens body of crosslinked acrylic material comprising copolymers of methacrylate and acrylate esters which are relatively hard and relatively soft at body temperature, crosslinked with a diacrylate ester wherein the crosslinked acrylic material has a substantially tack-free surface, a crosslink density of between $1.2 \times 10^{-1}$ and $3.0 \times 10^{-1}$ moles per liter, a glass transition temperature between -30° and 25°C.

2. An IOL body according to claim 1 wherein the lens is formed by chemically crosslinking the diacrylate ester with a partially polymerized mixture of the copolymers, curing the crosslinked acrylic material and holding the cured crosslinked acrylic material at a temperature below its Beta-relaxation temperature while machining the lens body.

3. An IOL according to claim 1 or claim 2 comprising:

   a tensile modulus between 70.3 kg cm$^{-2}$ (1000 psi) and 210.9 kg cm$^{-2}$ (3000 psi) and an elongation at break of at least 100%.

4. An IOL body according to any one of the preceeding claims wherein the relatively hard methacrylate ester is a fluoroacrylate.

5. An IOL body according to any one of the preceeding claims, wherein the copolymer comprises ethyl methacrylate, trifluoro ethyl methacrylate and an acrylate ester present in percent be weight concentration of 25 to 45, 5 to 25 and 30 to 60%, respectively.

6. An IOL body according to any one of the preceeding claims wherein the acrylate ester comprises n-butyl acrylate, ethyl acrylate or 2-ethyl hexyl acrylate.

7. An IOL body according to any one of the preceeding claims wherein the diacrylate ester is present in a percent by weight concentration of 2.5 to 6.0%.

8. An IOL body according to any one of the preceding claims wherein the diacrylate ester comprises ethylene glycol dimethacrylate, propylene glycol dimethacrylate or ethylene glycol diacrylate.

9. A method of forming a deformable-elastic intraocular lens body comprising the steps of:
   (a) mixing copolymers of methacrylate and acrylate esters which are relatively hard and relatively soft at body temperature;
   (b) partially polymerizing the product of Step (a);
   (c) chemically crosslinking the product of Step (b) with a diacrylate ester;
   (d) curing the product of Step (c); and
   (e) forming a lens body having a predetermined optical characteristic from the product of Step (d).

10. A method according to claim 9 wherein Step (e) comprises holding the product of Step (d) at a temperature below its Beta-relaxation temperature while machining the lens body.

11. The method according to claim 9 or claim 10 wherein the methacrylate and acrylate esters are mixed together in approximately a 45 to 55% by weight ratio.

12. A method according to any one of claims 9 to 11 wherein the diacrylate ester of Step (c) is present in a percent composition by weight of 2.5 to 6.0%.

13. A method according to any one of claims 9 to 12 further including the mixing of UV-absorber and a free radical initiator in Step (a).

14. A method according to any one of claims 9 to 13 wherein the relatively hard methacrylate ester is a fluoroacrylate.

15. A method according to any one of claims 9 to 14 wherein Step (a) further includes mixing the fluoroacrylate in a concentration range by weight of between 5 and 25% with ethyl methacrylate in a concentration range by weight of between 25 and 45% and an acrylate ester selected from the group consisting of n-butyl acrylate, ethylacrylate and 2-ethyl hexyl acrylate in a concentration range by weight of between 30 and 60%.

16. A method according to any one of claims 9 to 15 wherein the fluoracrylate is trifluoro ethyl methacrylate.

17. A method according to any one of claims 13 to 16 wherein UV-absorber is mixed in at a concentration range by weight of between 0 and 10% and the free radical initiator is mixed in at a concentration range by weight of 0.05 and 0.2%.

18. A deformable intraocular lens comprising a lens body as claimed in any one of claims 1 to 8 or when produced by the method of claims 9 to 17 together and flexible haptics attached to the lens body for positioning the lens body in the eye.

19. An IOL according to claim 18 wherein each haptic is attached by forcing an enlarged end thereof into a smaller hole in an edge of the lens body.

FIG. 1

FIG. 2

FIG. 3

FIG. 4

FIG. 5

FIG. 6

STIFFNESS

GLASS
STATE

LEATHER
STATE

RUBBERY STATE

CROSS-
LINKED
RUBBER STATE.

$T_g$

LIQUID

TEMPERATURE

10

EP 0 514 096 A2

EMA | 34

nBA/EA | 52

TFEMA | 10

UV-2098 | 1.5

USP-245 | 0.05

MIX DEAREATE →

OVEN 60°C 2hrs. →

SYRUP COOL TO 25°C | 97.5

EGDMA | 2.5

USP-245 | 0.1

MIX DEAREATE →

POUR INTO MOLD (FIG.8) →

OVEN 60°C 16hrs →

OVEN 90°C 24hrs

↓

COOL TO 25°C

LEGEND:

EA = ETHYL ACRYLATE
EMA = ETHYL METHACRYLATE
nBA = n BUTYLACRYLATE
TFEMA = TRIFLUOROETHYL METHACRYLATE
UV-2098 = 2-HYDROXY 4-ETHOXYACRYLOXY BENZOPHENONE
USP-245 = FREE RADICAL INITIATOR
EDMA = ETHYLENE GLYCOL DIMETHACRYLATE

FIG. 7

MOLD No. 2

MOLD No. 1

FIG. 8

FIG. 9